# EUROPÄISCHE PATENTSCHRIFT

(11) **EP 3 989 917 B1**
(45) Veröffentlichungstag und Bekanntmachung des Hinweises auf die Patenterteilung: **15.10.2025**
(21) Anmeldenummer: 20731841.1
(22) Anmeldetag: 08.06.2020
(51) Int. Cl.: A61K 8/11, A61K 8/06, A61K 8/20, A61K 8/73, A61K 8/37, A61K 8/44, A61K 8/46, A61K 8/55, A61Q 19/00

(54) **CALCIUMALGINATPARTIKEL**
CALCIUM ALGINATE PARTICLE
PARTICULES D'ALGINATE DE CALCIUM

(30) Priorität: 28.06.2019 DE 102019209475
(43) Veröffentlichungstag der Anmeldung: 04.05.2022
(73) Patentinhaber: Beiersdorf AG, 22529 Hamburg (DE)
(72) Erfinder: BURR, Franziska, 22765 Hamburg (DE); ROSENBAUM, Sebastian, 22769 Hamburg (DE); VOLBRICH, Heike, 22303 Hamburg (DE); RASCHKE, Thomas, 25421 Pinneberg (DE)
(74) Vertreter: Beiersdorf AG
(86) Internationale Anmeldenummer: PCT/EP2020/065766
(87) Internationale Veröffentlichungsnummer: WO 2020/259989

(56) Entgegenhaltungen:
- EP-A1- 1 413 298
- US-A1- 2014 370 098

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Der Stand der Technik kennt verschiedene kosmetische Zubereitungen zur Pflege der menschlichen Haut. So werden beispielsweise in WO 2015/169456 A1 kosmetische Zubereitungen offenbart, welche eine Gelphase aufweisen, in der Perlen, im wesentlichem kugelförmige Partikel mit einer Größe von 0,1 bis 20 mm, suspendiert sind. Die im wesentlichen kugelförmigen Partikel sind erhältlich, indem eine Emulsion mit Alginat (Algin) versetzt wird und die Emulsion anschließend in eine Lösung mit Calciumionen getropft wird. Die Interaktion des Alginats und der Calciumionen führt zu einem sofortigen und vollständigen Gelieren des Tropfens. Die so erhaltenen im wesentlichen kugelförmigen Partikel können entnommen und in einer Gelphase suspendiert werden kann. Der wesentliche Vorteil bei der Verwendung derartiger Partikel in kosmetischen Zubereitungen ist, dass in die Partikel Wirkstoffe eingebracht werden können, welche ggf. in dem umliegenden Gel nicht stabil wären.

Gemäß WO 2015/169456 A1 ist die kosmetische Zubereitung in einem Schleppkolbenspender enthalten. Bei Entnahme werden die kugelförmigen Partikel zerkleinert bzw. aufgebrochen und mit dem umfliegenden Gel vermengt, so dass eine homogene kosmetische Zubereitung enthalten wird und die Wirkstoffe freigegeben werden.

Nachteilig am den offenbarten kugelförmigen Partikeln bzw. Perlen aus WO 2015/169456 A1 ist der Umstand, dass diese Silikonöle, insbesondere Cyclomethicone, enthalten. Dieser Inhaltstoff ist von vielen Verbrauchern trotz seiner vorteilhaften Wirkweise oftmals nicht mehr erwünscht. Folglich werden in kosmetischen Produkten zunehmend Silikonöle ausformuliert.

Jedoch wird der Fachmann beim Austausch von Silikonölen vor unvorhergesehene Probleme gestellt, die basierend auf seinem Fachwissen nicht zu lösen sind. Werden beispielsweise anstelle von Silikonölen, Öle auf Basis von Estern zur Ausbildung der Alginatbasierten, im wesentlichen kugelförmigen Partikel auf Emulsionsbasis verwendet, kommt es beim beschriebenen Eintropfen der Emulsion in eine wässrige Lösung mit Calciumionen zur Ausbildung der Partikel zu einem Ausbluten der Emulsionskomponenten in die vorgelegt wässrige Lösung. Das bedeutet, dass sich die Lösung enthaltend die Calciumionen eintrübt. Die Ursache dafür ist, dass vermehrt Komponenten aus der Emulsion in die Lösung gelangen, statt dass diese Komponenten in den gebildeten Partikeln gebunden werden. Werden die Partikel aus der Lösung anschließend entnommen, um sie in eine kosmetische Zubereitung einzuarbeiten, wird die ursprünglich eingesetzte Emulsion nicht mehr vollständig umgesetzt, so dass der Verlust durch das Ausbluten in die Lösung die Produktionskosten steigen lässt.

Aufgabe der vorliegenden Erfindung war es daher, die die beschrieben Nachteile des Standes der Technik zu überwinden.

Weiterhin kennt der Fachmann das Dokument EP 1413298 A1 welches mit Calciumalginat umhüllte Makrokapseln offenbart, die über ein Tropfverfahren hergestellt werden. Jedoch konnte auch dieses Dokument keinen Hinweis auf die vorliegende Erfindung geben.

Überraschend wurde nun gefunden, dass der Gegenstand der vorliegenden Erfindung nicht die Nachteile des Standes der Technik aufweist.

Gegenstand der vorliegenden Erfindung ist ein Verfahren zur Herstellung von Calciumalginatpartikeln enthaltend eine Emulsion, umfassend die folgenden Verfahrensschritte:
i. Bereitstellung einer silikonfreien Öl-in-Wasser Emulsion umfassend
   a. Natriumalginat;
   b. einer Ölphase enthaltend mindestens ein Esteröl, und
   c. mindestens ein anionisches Tensid aufweisend eine Alkylkette mit mehr als 12 Kohlenstoffatomen;
ii. Bereitstellung einer wässrigen Lösung enthaltend Calciumchlorid
iii. Eintropfen der silikonfreien Öl-in-Wasser Emulsion in die wässrige Lösung zur Ausbildung der Calciumalginatpartikel.

Ein weiterer Gegenstand der vorliegenden Erfindung ist ein kosmetisches Produkt enthaltend die Calciumalginatpartikel hergestellt nach dem erfindungsgemäßen Verfahren.

Ein weiterer Gegenstand der vorliegenden Erfindung sind die Calciumalginatpartikel hergestellt nach dem erfindungsgemäßen Verfahren.

Bei Eintropfen in eine wässrige Lösung enthaltend mindestens ein Calciumsalz zeigt die erfindungsgemäße Emulsion überraschend kein Ausbluten.

Sollten Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der Öl-in-Wasser Emulsion.

Sollten Verhältnisse von Komponenten/Substanzen/Stoffgruppen offenbart werden, so beziehen sich diese Verhältnisse auf Gewichtsverhältnisse der genannten Komponenten/Substanzen/Stoffgruppen.

Die erfindungsgemäß verwendete Öl-in-Wasser Emulsion, nachfolgend lediglich als Emulsion bezeichnet, enthält erfindungsgemäß Natriumalginat. Vorteilhaft ist es, wenn der Anteil von Natriumalginat in der Emulsion von 0,2 bis 3 Gew.-%, bevorzugt von 0,5 bis 2 Gew.-% und insbesondere bevorzugt von 0,75 bis 1,5 Gew.-% beträgt, wobei sich die Angaben auf das Gesamtgewicht der Emulsion beziehen.

Vorteilhaft beträgt der Anteil der Ölphase in der Emulsion von 10 bis 25 Gew.-%, bevorzugt von 12 bis 20 Gew.-% und insbesondere von 14 bis 18 Gew.-%, wobei sich die Angaben auf das Gesamtgewicht der Emulsion beziehen und Tenside und Emulgatoren definitionsgemäß nicht zu Ölphase gerechnet werden.

Unter Emulgatoren und Tensiden werden definitionsgemäß alle Substanzen verstanden, welche im International Cosmetic Ingredient Dictionary and Handbook, Thirteenth Edition 2010, (ISBN 1-882621-47-6) unter der Bezeichnung "emulsifying agent und/oder surfactant " geführt werden, sofern die jeweiligen Substanzen in dieser Offenbarung nicht einer anderen Stoffklasse zugeordnet werden.

Die Ölphase der eingesetzten Emulsion umfasst mindestens ein Esteröl. Unter Esterölen werden unter normalbedingen flüssige Ester bezeichnet, welche nicht mit Wasser mischbar sind. Vorteilhaft sind die erfindungsgemäßen Esteröle gewählt aus der Gruppe der Triglyceriden von linearen oder verzweigten, gesättigten oder ungesättigten, gegebenenfalls hydroxylierten C8-30-Fettsäuren sowie Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, sofern diese unter Normalbedingungen flüssig sind. Bevorzugte bei 20°C flüssige Esteröle sind gewählt aus der Gruppe Isopropylpalmitat, Isopropylstearat, Isodecyl Neopentanoate, Cetearylisononanoate, C12-15 Alkylbenzoat, Ethylhexylstearat, Caprylic Capric Triglyceride, Cocoglycerides Octylcocoate, Dibutyladipate und Octylpalmitate. Es ist insbesondere bevorzugt, wenn als Esteröl mindestens Isodecyl Neopentanoate enthalten ist.

Es ist insbesondere vorteilhaft, wenn der Anteil der Esteröle von 10 bis 25 Gew.-%, bevorzugt von 12 bis 20 Gew.-% und insbesondere von 14 bis 18 Gew.-% bezogen auf das Gesamtgewicht der Emulsion beträgt.

Weiterhin ist es vorteilhaft, wenn die Ölphase der Emulsion natürliche Öle umfasst, insbesondere vorteilhaft Sonnenblumen-, Soja- Maiskeim-, Mandel-, Oliven- und/oder Rapsöl.

Ferner enthält die erfindungsgemäß eingesetzte Emulsion mindestens ein anionisches Tensid aufweisend eine Alkylkette mit mehr als 12 Kohlenstoffatomen. Derartige bevorzugte Tenside sind dadurch gekennzeichnet, dass diese gewählt sind aus der Gruppe der Glutamate, Citrate, Phosphate und Sulfate.

Erfindungsgemäß vorteilhaft zu wählende Tenside sind insbesondere Sodium Stearoyl Glutamate und/oder Sodium Cetearyl Sulfate und/oder Kalium Cetylphosphate, wobei der Einsatz von Sodium Stearoyl Glutamate insbesondere bevorzugt ist.

Vorteilhaft beträgt der Anteil der anionischen Tenside aufweisend eine Alkylkette mit mehr als 12 Kohlenstoffatomen von 0,01 bis 2,0 Gew.-%, bevorzugt von 0,04 bis 0,9 Gew.-% und insbesondere bevorzugt von 0,05 bis 0,6 Gew.-%, bezogen auf das Gesamtgewicht der Emulsion.

Es ist insbesondere vorteilhaft, wenn der Anteil von Sodium Stearoyl Glutamate und/oder Sodium Cetearyl Sulfate von 0,01 bis 0,8 Gew.-%, bevorzugt von 0,04 bis 0,4 Gew.-% und insbesondere bevorzugt von 0,05 bis 0,2 Gew.-%, bezogen auf das Gesamtgewicht der Emulsion, beträgt.

Vorteilhaft umfasst die erfindungsgemäß verwendete Emulsion keine nichtionischen Emulgatoren oder Tenside.

Weiterhin ist es vorteilhaft, wenn die erfindungsgemäß verwendete Emulsion keine anionischen Tenside aufweisend eine Alkylkette mit 12 oder weniger Kohlenstoffatomen enthält.

Ferner ist es vorteilhaft, wenn die Emulsion Methylpropandiol, bevorzugt in einem Anteil von 1 bis 6 Gew.-%, enthält, wobei sich die Gewichtsprozentangabe auf das Gesamtgewicht der Emulsion bezieht.

Weiterhin ist es vorteilhaft, wenn die Emulsion Phenoxyethanol umfasst, wobei der Anteil von Phenoxyethanol bevorzugt von 0,2 bis 1 Gew.-% beträgt, bezogen auf das Gesamtgewicht der Emulsion.

Vorteilhafte Emulsionen sind ferner dadurch gekennzeichnet, dass diese Hexandiol, insbesondere 1,2-Hexandiol enthalten, wobei der Anteil von Hexandiol, bzw. der Anteil von 1,2-Hexandiol, bevorzugt von 0,3 bis 1,5 Gew.-%, bezogen auf das Gesamtgewicht der Emulsion, beträgt.

Weiterhin ist es vorteilhaft gemäß der vorliegenden Erfindung, wenn die Emulsion Wasser in einem Anteil von mindestens 70 Gew.-% umfasst. Vorteilhaft beträgt der Anteil von Wasser von 70 Gew.-% bis 80 Gew.-%, bezogen auf das Gesamtgewicht der Emulsion.

Weiterhin ist es vorteilhaft, wenn die Emulsion der vorliegenden Erfindung weitere Wirkstoffe gewählt aus der Gruppe bestehend aus Glycyrrhetinsäure, Arctiin, Folsäure, Coenzym Q10 (Ubiquinon), alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, Vitamin C, Vitamin C phosphate, Vitamin C palmitate, Niacinamide, Vitamin A palmitate, Retinol, Panthenol, Glycyrrhiza Inflata Wurzelextrakt, Arctiin, Licochalcon A, 4-Butylresocinol, N-[(2,4-Dihydroxyphenyl)thiazol-2-yl]isobutyramide, Honociol und Magnolol (auch als Bestandteil von Magnolia-Extrakten), Hyaluronsäure und/oder Silymarin (Mariendistelextrakt) umfasst.

Weiterhin ist es vorteilhaft, wenn die Emulsion der vorliegenden Erfindung weitere UV-A-, UV-B- und/oder Breitbandfiltersubstanz enthalten. Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate).

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex^{®} 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Symrise erhältlich ist;
- 1,4-di(2-oxo-10-Sulfo-3-bornylidenmethyl)-Benzol (auch: 3,3'-(1,4-Phenylendimethy-lene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
   Vorteilhafte Breitbandfilter sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb^{®} S bei der BASF erhältlich ist.

Die erfindungsgemäße Emulsion ist weiterhin dadurch gekennzeichnet, dass diese frei von Silikonölen ist. Folglich sind keine Öle enthaltend Polydimethylsiloxaneinheiten enthalten.

Weiterhin ist es vorteilhaft, wenn die Emulsion dadurch gekennzeichnet ist, dass kein Mineralöl enthalten ist.

Die erfindungsgemäß eingesetzte wässrige Lösung enthaltend Calciumchlorid ist vorteilhaft dadurch gekennzeichnet, dass diese Calciumchlorid in einem Gesamtanteil von 0,5 bis 5 Gew.-%, bevorzugt 1 bis 4 Gew.-% und insbesondere bevorzugt von 1,5 bis 3 Gew.-% enthält, wobei sich die Gew.-% Angaben auf das Gesamtgewicht der wässrigen Lösung beziehen.

Weiterhin umfasst die erfindungsgemäße wässrige Lösung vorteilhaft 1,2-Hexandiol. Enthält die wässrige Lösung 1,2-Hexandiol, so ist es erfindungsgemäß von Vorteil, wenn der Anteil von 1,2-Hexandiol von 0,5 bis 2 Gew.-% beträgt, bezogen auf das Gesamtgewicht der wässrigen Lösung.

Ferner ist es die vorteilhaft, wenn die wässrige Lösung Methylpropandiol enthält, wobei der Anteil von Methylpropandiol bevorzugt im Bereich von 1 Gew.-% bis 5 Gew.-% bezogen auf das Gesamtgewicht der wässrigen Lösung beträgt.

Es ist ebenfalls vorteilhaft, wenn die wässrige Lösung Phenoxyethanol enthält, wobei der Anteil von Phenoxyethanol bevorzugt im Bereich von 0,5 Gew.-% bis 1 Gew.-% bezogen auf das Gesamtgewicht der wässrigen Lösung beträgt.

Es wurde überraschend gefunden, dass bei Anwendung des erfindungsgemäßen Verfahrens die Trübung in der wässrigen Phase bei Herstellung der Partikel geringer ist, als bei Anwendung eines nicht erfindungsgemäßen Verfahrens. Dieses wird anhand der Vergleichsversuche in den Beispielen verdeutlicht.

Es ist erfindungsgemäß vorteilhaft, wenn nach dem Eintropfen der Emulsion in die wässrige Lösung die erhaltenen Partikel mindestens 30 Minuten in dem Bad verweilen, so dass eine Aushärtung erreicht wird. Folglich werden die Partikel strukturell stabiler und lassen sich nicht so leicht aufbrechen und/oder zerkleinern.

Weiterhin ist es vorteilhaft, wenn die Partikel nach Herstellung aus der Lösung entnommen werden, bspw. vorteilhaft durch Sieben, und folglich zur Herstellung eines kosmetischen Produktes in einer kosmetischen Zubereitung suspendiert werden.

Es ist ebenfalls vorteilhaft, wenn die Partikel vor Suspendierung in einer kosmetischen Zubereitung mit vollentsalztem Wasser gewaschen werden.

Die Herstellung und Suspendierung erfolgt vorteilhaft bei einer Temperatur von 10 bis 30°C, wobei sich die Temperaturangabe auf die Außentemperatur und die Temperatur der Emulsion sowie der wässrigen Lösung bezieht.

Die erhaltenen Partikel weisen vorteilhaft einen Durchmesser von 0,1 bis 20 mm, bevorzugt 0,5 bis 5 mm auf. Der Durchmesser der Partikel lässt sich durch Messen mit einer Schieblehre oder durch sieben ermitteln.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die erfindungsgemäßen kugelförmigen Partikel wurden wie folgt hergestellt:
Zunächst wurden die folgenden Emulsionen bereitgestellt, wobei die Beispiele E.1 und E.2 erfindungsgemäße Emulsionen sind, während E.3 bis E.5 nicht erfindungsgemäße Vergleichsbeispiele darstellen:

| **Inhaltstoff** | **E.1** | **E.2** | **E.3** | **E.4** | **E.5** |
|---|---|---|---|---|---|
| Sodium Alginate | 1 | 1 | 1 | 1 | 1 |
| Aqua | Ad 100 | Ad 100 | Ad 100 | Ad 100 | Ad 100 |
| Cocoglycerides | 6 | 6 | 6 | 6 | 6 |
| Isodecyl Neopentanoate | 10 | 10 | 10 | 10 | 10 |
| 1,2- Hexandiol | 1 | 1 | 1 | 1 | 1 |
| Methylpropandiol | 4 | 4 | 4 | 4 | 4 |
| Phenoxyethanol | 0,8 | 0,8 | 0,8 | 0,8 | 0,8 |
| Sodium Stearoyl Glutamate | 0,3 - | | | | |
| Sodium Cetearyl Sulfate | | 0,3 | | | |
| Polyglyceryl-3 Methylglucose Distearate | | | 0,3 | | |
| Sodium Laureth Sulfate | | | | 0,3 | |
| Polyglyceryl-10 Stearate | | | | | 0,075 |

Zudem wurde eine wässrige Lösung mit den folgenden Inhaltsstoffen bereitgestellt:

| **Inhaltsstoffe** | **Wässrige Lösung** |
|---|---|
| Aqua | Ad 100 |
| 1,2-Hexandiol | 1 |
| Methylpropanediol | 4 |
| Phenoxyethanol | 0,8 |
| Calciumchlorid | 2 |

Zur Herstellung wurde die jeweilige Emulsion unter Normalbedingungen mittels eines Scheidetrichters in einen Becher getropft, der 250 ml der wässrigen Lösung enthielt. Es wurden jeweils 50 ml der Emulsion in den Becher getropft. Bei jeder Probe haben sich Calciumalginatpartikel enthaltend die Emulsion gebildet.

Nach Aussieben der gebildeten Partikel wurde die Lösung aus dem Becher aufgeschüttelt, bis eine homogene Zusammensetzung erhalten wurde. In aufgeschüttelter Form wurde sofort ein Transmissionspektrum bei 400 nm mit einer 1 cm Küvette und einem Spalt von 2 nm gegen Wasser als Referenz aufgenommen. Die Messwerte sind nachfolgend aufgeführt:

| Probe | Enthaltenes Tensid | Transmission bei 400nm |
|---|---|---|
| **E.1** | Sodium Stearoyl Glutamate | 97 % |
| **E.2** | Sodium Cetearyl Sulfate | 67 % |
| **E.3** | Polyglyceryl-3 Methylglucose Distearate | 18% |
| **E.4** | Sodium Laureth Sulfate | 17% |
| **E.5** | Polyglyceryl-10 Stearate | 2% |

Überraschend wurde gefunden, dass bei Verwendung der erfindungsgemäßen Tenside eine deutlich geringere Trübung verdeutlicht durch eine hohe Transmission eingetreten ist. Folglich konnte das Ausbluten bei der Herstellung verhindert werden. Nicht ionische Tenside/Emulgatoren sind ungeeignet, um ein Ausbluten zu verhindern (Siehe E.3 und E.5). Auch andere anionische Tenside sind ungeeignet (siehe E.4).

Weitere Emulsionen, welche erfindungsgemäß verwendet werden können sind nachstehend aufgeführt.

| **Inhaltstoff** | **E.6** | **E.7** | **E.8** | **E.9** | **E.10** |
|---|---|---|---|---|---|
| Sodium Alginate | 1 | 1 | 1 | 1 | 1 |
| Aqua | Ad 100 | Ad 100 | Ad 100 | Ad 100 | Ad 100 |
| Cocoglycerides | | 5 | 16 | 6 | 6 |
| Isodecyl Neopentanoate | | | | 10 | 10 |
| Caprylic/Capric Triglyceride | 4 | 3 | 8 | | |
| Ethylhexylstearat | 4 | | | | |
| Isopropylpalmitat | 8 | 8 | 8 | | |
| Sonnenblumenöl | 0,5 | | | 0,5 | |
| 1,2- Hexandiol | 1 | 1 | 1 | 1 | 1 |
| Methylpropandiol | 4 | 4 | 4 | 4 | 4 |
| Phenoxyethanol | 0,8 | 0,8 | 0,8 | 0,8 | 0,8 |
| Sodium Stearoyl Glutamate | 0,3 | 0,3 | | 0,3 | 0,3 |
| Sodium Cetearyl Sulfate | | | 0,3 | | |
| Kalium Cetylphosphate | | | | | 1,5 |
| Ubichinone | | | | 0,1 | |
| Klettenfruchtextrakt (Arctiin) | | | | | 0,3 |
| Ascorbylpalmitat | | | | | 0,1 |
| Kreatin | | 0,3 | | | |
| Magnolol | | | | | 0,1 |
| TiO₂ | | | | 0,5 | |
| Bis-Ethylhexyloxyphenol Methoxyphenyl Triazine | 1 | | 0,5 | | |
| Butyl Methoxydibenzoylmethane | | 1 | 0,5 | | |
| Octocrylene | 3 | | 2 | | |
| Ethylhexyl Salicylate | | 3 | | | |
| Homosalate | | | 2 | | |
| Octyl Methoxycinnamate | 1 | | | | |

Die jeweilige Emulsion E.6 bis E.10werden unter Normalbedingungen mittels eines Scheidetrichters in einen Becher getropft, der 250 ml der oben beschriebenen wässrigen Lösung befüllt ist. Es wurden jeweils 50 ml der Emulsion in den Becher getropft. Bei jeder Probe haben sich Calciumalginatpartikel enthaltend die Emulsion gebildet.

## Patentansprüche

1. Verfahren zur Herstellung von Calciumalginatpartikeln enthaltend eine Emulsion, umfassend die folgenden Verfahrensschritte:
i. Bereitstellung einer silikonfreien Öl-in-Wasser Emulsion umfassend
a. Natriumalginat;
b. einer Ölphase enthaltend mindestens ein Esteröl,
c. mindestens ein anionisches Tensid aufweisend eine Alkylkette mit mehr als 12 Kohlenstoffatomen;
ii. Bereitstellung einer wässrigen Lösung enthaltend Calciumchlorid
iii. Eintropfen der silikonfreien Öl-in-Wasser Emulsion in die wässrige Lösung zur Ausbildung der Calciumalginatpartikel.

2. Verfahren nach Anspruch 1 **dadurch gekennzeichnet, dass** der Anteil von Natriumalginat in der Emulsion von 0,2 bis 3 Gew.-%, bevorzugt von 0,5 bis 2 Gew.-% und insbesondere bevorzugt von 0,75 bis 1,5 Gew.-% beträgt, wobei sich die Angaben auf das Gesamtgewicht der Emulsion beziehen.

3. Verfahren nach einem der vorhergehenden Ansprüche **dadurch gekennzeichnet, dass** der Anteil der Ölphase in der Emulsion von 10 bis 25 Gew.-%, bevorzugt von 12 bis 20 Gew.-% und insbesondere von 14 bis 18 Gew.-%, wobei sich die Angaben auf das Gesamtgewicht der Emulsion beziehen und Tenside und Emulgatoren definitionsgemäß nicht zu Ölphase gerechnet werden.

4. Verfahren nach einem der vorhergehenden Ansprüche **dadurch gekennzeichnet, dass** mindestens eine Esteröl gewählt aus der Gruppe der Triglyceriden von linearen oder verzweigten, gesättigten oder ungesättigten, gegebenenfalls hydroxylierten C8-30-Fettsäuren sowie der Ester von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, sofern diese unter Normalbedingungen flüssig sind.

5. Verfahren nach einem der Ansprüche 1 bis 3 **dadurch gekennzeichnet, dass** das mindestens eine Esteröl gewählt ist aus der Gruppe Isopropylpalmitat, Isopropylstearat, Isodecyl Neopentanoate, Cetearylisononanoate, C12-15 Alkylbenzoat, Ethylhexylstearat, Caprylic Capric Triglyceride, Octylcocoate, Cocoglycerides, Dibutyladipate und Octylpalmitate.

6. Verfahren nach einem der vorhergehenden Ansprüche **dadurch gekennzeichnet, dass** der Anteil der Esteröle von 10 bis 25 Gew.-%, bevorzugt von 12 bis 20 Gew.-% und insbesondere von 14 bis 18 Gew.-% bezogen auf das Gesamtgewicht der Emulsion beträgt.

7. Verfahren nach einem der vorhergehenden Ansprüche **dadurch gekennzeichnet, dass** die Ölphase der Emulsion natürliche Öle umfasst, insbesondere vorteilhaft Sonnenblumen-, Soja- Maiskeim-, Mandel-, Oliven- und/oder Rapsöl.

8. Verfahren nach einem der vorhergehenden Ansprüche **dadurch gekennzeichnet, dass** das mindestens eine anionische Tensid aufweisend eine Alkylkette mit mehr als 12 Kohlenstoffatomen gewählt ist aus der Gruppe der Glutamate, Citrate, Phosphate und Sulfate.

9. Verfahren nach einem der vorhergehenden Ansprüche **dadurch gekennzeichnet, dass** das mindestens eine anionische Tensid aufweisend eine Alkylkette mit mehr als 12 Kohlenstoffatomen gewählt ist aus Sodium Stearoyl Glutamate und/oder Sodium Cetearyl Sulfate und/oder Kalium Cetylphosphate, wobei der Einsatz von Sodium Stearoyl Glutamate insbesondere bevorzugt ist.

10. Verfahren nach einem der vorhergehenden Ansprüche **dadurch gekennzeichnet, dass** der Anteil der anionischen Tenside aufweisend eine Alkylkette mit mehr als 12 Kohlenstoffatomen von 0,01 bis 2,0 Gew.-%, bevorzugt von 0,04 bis 0,9 Gew.-% und insbesondere bevorzugt von 0,05 bis 0,6 Gew.-%, bezogen auf das Gesamtgewicht der Emulsion.

11. Verfahren nach einem der vorhergehenden Ansprüche **dadurch gekennzeichnet, dass** die Emulsion frei von Mineralölen ist.

12. Verfahren nach einem der vorhergehenden Ansprüche **dadurch gekennzeichnet, dass** die wässrige Lösung enthaltend Calciumchlorid **dadurch gekennzeichnet ist, dass** diese Calciumchlorid in einem Gesamtanteil von 0,5 bis 5 Gew.-%, bevorzugt 1 bis 4 Gew.-% und insbesondere bevorzugt von 1,5 bis 3 Gew.-% enthält, wobei sich die Gew.-% Angaben auf das Gesamtgewicht der wässrigen Lösung beziehen.

13. Partikel hergestellt gemäß einem der Ansprüche 1 bis 12.

14. Partikel nach Anspruch 13 **dadurch gekennzeichnet, dass** diese einen Durchmesser von 0,1-20 mm, bevorzugt 0,5-5 mm aufweisen.

15. Kosmetisches Produkt enthaltend die Partikel hergestellt gemäß einem der Ansprüche 1 bis 12.

## Claims

1. Process for producing calcium alginate particles containing an emulsion, comprising the following process steps:
i. providing a silicone-free oil-in-water emulsion comprising
a. sodium alginate;
b. an oil phase containing at least one ester oil,
c. at least one anionic surfactant having an alkyl chain with more than 12 carbon atoms;
ii. providing an aqueous solution containing calcium chloride
iii. adding the silicone-free oil-in-water emulsion dropwise into the aqueous solution to form the calcium alginate particles.

2. Process according to Claim 1, **characterized in that** the proportion of sodium alginate in the emulsion is from 0.2% to 3% by weight, preferably from 0.5% to 2% by weight and especially preferably from 0.75% to 1.5% by weight, where the figures relate to the total weight of the emulsion.

3. Process according to either of the preceding claims, **characterized in that** the proportion of the oil phase in the emulsion from 10% to 25% by weight, preferably from 12% to 20% by weight and in particular from 14% to 18% by weight, where the figures relate to the total weight of the emulsion and surfactants and emulsifiers by definition are not considered to be part of the oil phase.

4. Process according to any of the preceding claims, **characterized in that** at least one ester oil selected from the group of triglycerides of linear or branched, saturated or unsaturated, optionally hydroxylated C8-30 fatty acids, and esters of linear or branched, saturated or unsaturated fatty alcohols with 2 - 30 carbon atoms with linear or branched, saturated or unsaturated fatty acids with 2 - 30 carbon atoms, provided that they are liquid under standard conditions.

5. Process according to any of Claims 1 to 3, **characterized in that** the at least one ester oil is selected from the group of isopropyl palmitate, isopropyl stearate, Isodecyl Neopentanoate, cetearyl isononanoate, C12-15 alkyl benzoate, ethylhexyl stearate, Caprylic/Capric Triglyceride, octyl cocoate, cocoglycerides, dibutyl adipate and octyl palmitate.

6. Process according to any of the preceding claims, **characterized in that** the proportion of the ester oils is from 10% to 25% by weight, preferably from 12% to 20% by weight and in particular from 14% to 18% by weight, based on the total weight of the emulsion.

7. Process according to any of the preceding claims, **characterized in that** the oil phase of the emulsion comprises natural oils, especially advantageously sunflower, soybean, maize germ, almond, olive and/or rapeseed oil.

8. Process according to any of the preceding claims, **characterized in that** the at least one anionic surfactant having an alkyl chain with more than 12 carbon atoms is selected from the group of glutamates, citrates, phosphates and sulfates.

9. Process according to any of the preceding claims, **characterized in that** the at least one anionic surfactant having an alkyl chain with more than 12 carbon atoms is selected from Sodium Stearoyl Glutamate and/or Sodium Cetearyl Sulfate and/or Potassium Cetyl Phosphate, with the use of Sodium Stearoyl Glutamate being especially preferred.

10. Process according to any of the preceding claims, **characterized in that** the proportion of the anionic surfactants having an alkyl chain with more than 12 carbon atoms from 0.01% to 2.0% by weight, preferably from 0.04% to 0.9% by weight and especially preferably from 0.05% to 0.6% by weight, based on the total weight of the emulsion.

11. Process according to any of the preceding claims, **characterized in that** the emulsion is free of mineral oils.

12. Process according to any of the preceding claims, **characterized in that** the aqueous solution containing calcium chloride is **characterized in that** it contains calcium chloride in a total proportion of from 0.5% to 5% by weight, preferably 1% to 4% by weight and especially preferably from 1.5% to 3% by weight, where the figures in % by weight relate to the total weight of the aqueous solution.

13. Particles produced according to any of Claims 1 to 12.

14. Particles according to Claim 13, **characterized in that** they have a diameter of 0.1-20 mm, preferably 0.5-5 mm.

15. Cosmetic product containing the particles produced according to any of Claims 1 to 12.

## Revendications

1. Procédé de préparation de particules d'alginate de calcium contenant une émulsion, comprenant les étapes de procédé suivantes :
i. mise à disposition d'une émulsion huile-dans-eau exempte de silicone comprenant
a. de l'alginate de sodium ;
b. une phase huileuse contenant au moins une huile d'ester,
c. au moins un tensioactif anionique présentant une chaîne alkyle comprenant plus de 12 atomes de carbone ;
ii. mise à disposition d'une solution aqueuse contenant du chlorure de calcium
iii. introduction goutte-à-goutte de l'émulsion huile-dans-eau exempte de silicone dans la solution aqueuse pour la formation des particules d'alginate de calcium.

2. Procédé selon la revendication 1, **caractérisé en ce que** la proportion d'alginate de sodium dans l'émulsion représente 0,2 à 3% en poids, de préférence 0,5 à 2% en poids et en particulier de préférence 0,75 à 1,5% en poids, les indications se rapportant au poids total de l'émulsion.

3. Procédé selon l'une des revendications précédentes, **caractérisé en ce que** la proportion de la phase huileuse dans l'émulsion 10 à 25% en poids, de préférence 12 à 20% en poids et en particulier 14 à 18% en poids, les indications se rapportant au poids total de l'émulsion et les tensioactifs et les émulsifiants n'étant par définition pas comptés dans la phase huileuse.

4. Procédé selon l'une des revendications précédentes, **caractérisé en ce qu'**au moins une huile d'ester choisie dans le groupe des triglycérides d'acides gras en C8-30 linéaires ou ramifiés, saturés ou insaturés, le cas échéant hydroxylés ainsi que des esters d'alcools gras linéaires ou ramifiés, saturés ou insaturés comprenant 2 à 30 atomes de carbone avec des acides gras linéaires ou ramifiés, saturés ou insaturés comprenant 2 à 30 atomes de carbone, pour autant que ceux-ci soient liquides dans les conditions normales.

5. Procédé selon l'une des revendications 1 à 3, **caractérisé en ce que** ladite au moins une huile d'ester est choisie dans le groupe palmitate d'isopropyle, stéarate d'isopropyle, néopentanoate d'isodécyle, isononanoate de cétéaryle, benzoate de C12-15-alkyle, stéarate d'éthylhexyle, triglycéride caprylique/caprique, cocoate d'octyle, cocoglycérides, adipate de dibutyle et palmitate d'octyle.

6. Procédé selon l'une des revendications précédentes, **caractérisé en ce que** la proportion des huiles d'esters représente 10 à 25% en poids, de préférence 12 à 20% en poids et en particulier 14 à 18% en poids, par rapport au poids total de l'émulsion.

7. Procédé selon l'une des revendications précédentes, **caractérisé en ce que** la phase huileuse de l'émulsion comprend des huiles naturelles, de manière particulièrement avantageuse l'huile de tournesol, l'huile de soja, l'huile de germes de maïs, l'huile d'amande, l'huile d'olive et/ou l'huile de colza.

8. Procédé selon l'une des revendications précédentes, **caractérisé en ce que** ledit au moins un tensioactif anionique présentant une chaîne alkyle comprenant plus de 12 atomes de carbone est choisi dans le groupe des glutamates, citrates, phosphates et sulfates.

9. Procédé selon l'une des revendications précédentes, **caractérisé en ce que** ledit au moins un tensioactif anionique présentant une chaîne alkyle comprenant plus de 12 atomes de carbone est choisi parmi le stéaroylglutamate de sodium et/ou le cétéarylsulfate de sodium et/ou le cétylphosphate de potassium, l'utilisation de stéaroylglutamate de sodium étant particulièrement préférée.

10. Procédé selon l'une des revendications précédentes, **caractérisé en ce que** la proportion des tensioactifs anioniques présentant une chaîne alkyle comprenant plus de 12 atomes de carbone 0,01 à 2,0% en poids, de préférence 0,04 à 0,9% en poids et en particulier de préférence 0,05 à 0,6% en poids, par rapport au poids total de l'émulsion.

11. Procédé selon l'une des revendications précédentes, **caractérisé en ce que** l'émulsion est exempte d'huiles minérales.

12. Procédé selon l'une des revendications précédentes, **caractérisé en ce que** la solution aqueuse contenant du chlorure de calcium est **caractérisée en ce que** celle-ci contient le chlorure de calcium en une proportion totale de 0,5 à 5% en poids, de préférence 1 à 4% en poids, en particulier de préférence de 1,5 à 3% en poids, les indications de % en poids se rapportant au poids total de la solution aqueuse.

13. Particules préparées selon l'une des revendications 1 à 12.

14. Particules selon la revendication 13, **caractérisées en ce que** celles-ci présentent un diamètre de 0,1-20 mm, de préférence de 0,5-5 mm.

15. Produit cosmétique contenant les particules préparées selon l'une des revendications 1 à 12.
